# EUROPÄISCHE PATENTANMELDUNG

(11) **EP 0 678 287 A2**
(43) Veröffentlichungstag der Anmeldung: **25.10.1995**
(21) Anmeldenummer: 95105885.8
(22) Anmeldetag: 20.04.1995
(51) Int. Cl.: A61F 6/00, A61F 6/04

(54) **Präservativ**

(30) Priorität: 21.04.1994 DE 4413879; 17.02.1995 DE 9505378 U
(71) Anmelder: Metz, Joachim, D-40472 Düsseldorf (DE)
(72) Erfinder: Metz, Joachim, D-40472 Düsseldorf (DE)
(74) Vertreter: Sparing - Röhl - Henseler Patentanwälte

(57) **Zusammenfassung**

Präservativ, dessen Handhabung dadurch erleichtert ist, daß es auf einen verdickten Ring aufgerollt ist, der nach dem Applizieren abfällt. Hierfür ist der Ring aufgeschnitten und entweder aus einem langgestreckten Material elastisch in die Ringform gebogen, oder aber er besteht aus mehreren Ringsegmenten.

## Beschreibung

Handelsübliche Präservative werden eingerollt verkauft. Beim Applizieren des Präservativs soll dieses abgerollt werden, was ziemliche Fingerfertigkeit voraussetzt.

Die existierenden Präservative sind also in ihrer Benutzung nicht problemlos.

Häufig kommt es zu überlangen Unterbrechungen des Liebesspiels: Nicht selten erfolgen Zwischenfälle bei der Anwendung, welche durch die nicht einfache Bestimmung der Ausrichtung des Präservativs, durch Abrutschen des Präservativs von der Peniskuppe und durch ähnliche Situationen verursacht werden. All diese Schwierigkeiten erfordern bei der Verwendung des Präservativs die volle Aufmerksamkeit des Benutzers, so daß nicht selten ein frühzeitiger Errektionsverlust und somit eine Unterbrechung des Liebesspiels die Folge ist.

Dadurch mißbilligt man oftmals die Anwendung eines Präservativs.

Die angeführten Probleme sollten in der heutigen Zeit der Aids-Problematik nicht mehr zu einer Ablehnung eines Präservativs führen. Im Gegenteil: Es sollte alles dafür getan werden, um die Kompliziertheiten bei der Verwendung eines Präservativs zu vermeiden.

Das im folgenden vorgestellte Präservativ verhindert die beschriebenen Nachteile.
1) leicht aufsetzbar,
2) totales Abrollen garantiert,
3) minimale Unterbrechung des Liebesspiels.

Die Problematik des bislang bekannten Präservativs ist nicht neu und es gibt eine Reihe von Vorschlägen, die die Handhabung vereinfachen sollen.

GB-A-2 253 352 offenbart einen Applikator in Form eines Ringes mit einer Nut, in die das aufgerollte Präservativ eingelegt ist. Mittels des Ringes soll das Präservativ appliziert werden; das aus einem Elastomer bestehende Präservativ rollt dabei relativ zu dem Ring, was zu Reibungsbelastung führt und das Präservativ beschädigen kann. Der Ring hat eine Sollbruchstelle, da er im Gegensatz zu dem Präservativ selbst nicht dehnbar ist. Da das Präservativ im Gebrauch mit elastischer Vorspannung anliegen muß, um nicht abzurutschen, im verpackten Zustand jedoch nicht vorgespannt ist, dürfte die richtige Bemaßung des Applikatordurchmessers problematisch sein.

Ein weiterer, ähnlicher Applikator ist in WO 88/02624 offenbart, bei dem die Mündung des Präservativs jedoch von dem Ring auf einen vergrößerten Durchmesser gespannt wird.

Ein Präservativ, das auf einen verdickten Ring aus elastischem Material aufgerollt ist, um die Handhabung zu erleichtern, ist aus US-A-5,163,448 bekannt. Der Ring ist deformierbar und in sich geschlossen, so daß der Nachteil besteht, daß er nach dem Applizieren des Präservativs wieder abgerollt werden muß.

Ein weiteres, mit einem Ring versehenes aufgerolltes Präservativ ist aus US-A-3,282,414 bekannt. Der hohle Ring umschließt den Rollwulst und enthält ein Gel, das beim Abrollen austritt; dabei rollt das Präservativ relativ zu dem Ring. Damit nach dem Applizieren der Ring entfernt werden kann, ist er aufgeschnitten und kann nach Abreißen einer ihn zusammenhaltenden Lasche geöffnet werden.

Die Erfindung beruht auf der Erkenntnis, daß ein Rollring, wie in der US-A-5,163,448 offenbart, zwar das Applizieren des Präservativs sehr erleichtert, jedoch den oben erwähnten Nachteil aufweist, daß der Ring nach der Applikation des Präservativs irgendwann wieder abgerollt werden muß. Deshalb ist es die Aufgabe der Erfindung, ein auf einen verdickten Ring aufgerolltes Präservativ zu schaffen, von dem der Ring nach dem Applizieren einfach abfällt.

Die vorliegende Erfindung geht von der Erkenntnis aus, daß die Handhabung von Präservativen zu wünschen übrigläßt und sucht nach einer Verbesserung, die möglichst auch kostengünstig ist.

Die erfindungsgemäß vorgesehene Lösung dieser Aufgabe ergibt sich aus dem Patentanspruch 1. In einer ersten Ausführung wird aus einem geradlinig hergestellten elastischen Material (z.B. Moosgummi oder dergleichen) ein entsprechend langes Stück vor dem Einrollen in das Präservativ in eine Ringform an den Präservativrand um das Präservativ gebogen. Nach dem Abrollen nimmt dieses in Ringform gebogene Material wieder seine langgestreckte Form an und fällt somit ohne weiteres Dazutun von alleine ab.

Alternativ kann man die Ringform auch aus mehreren, z.B. zwei oder drei Sektoren, erhalten.

Bei nur zwei Sektoren sollten diese ebenfalls aus einem geraden Strang gefertigt werden, der erst vor dem Einrollen in die gebogene Form gebracht wird. Bei drei oder mehr Sektoren kann man diese direkt in der Sektorform fertigen, da sie nach dem Applizieren des Präservativs an diesem keinen Halt mehr haben und abfallen.

Ausführungsbeispiele sind in der beigefügten Zeichnung dargestellt und werden nachstehend erläutert.
- Fig. 1: zeigt das in Längsform hergestellte Stück Material, welches in seiner Anwendung dann zu einem Ring gebogen wird.
- Fig. 2: zeigt ein entrolltes Präservativ gemäß der Erfindung.
- Fig. 3: ist eine Draufsicht auf das in die Ringform gebogene Material, bestehend aus einem Stück.
- Fig. 4: ist eine Draufsicht auf das in die Ringform gebogene Material, bestehend aus mehreren Stückteilen.
- Fig. 5: zeigt eine mögliche Abwandlung der Oberflächenstruktur des zu verwendenden Materials.
- Fig. 6: zeigt eine weitere mögliche Abwandlung der Oberflächenstruktur des zu verwendenden Materials.

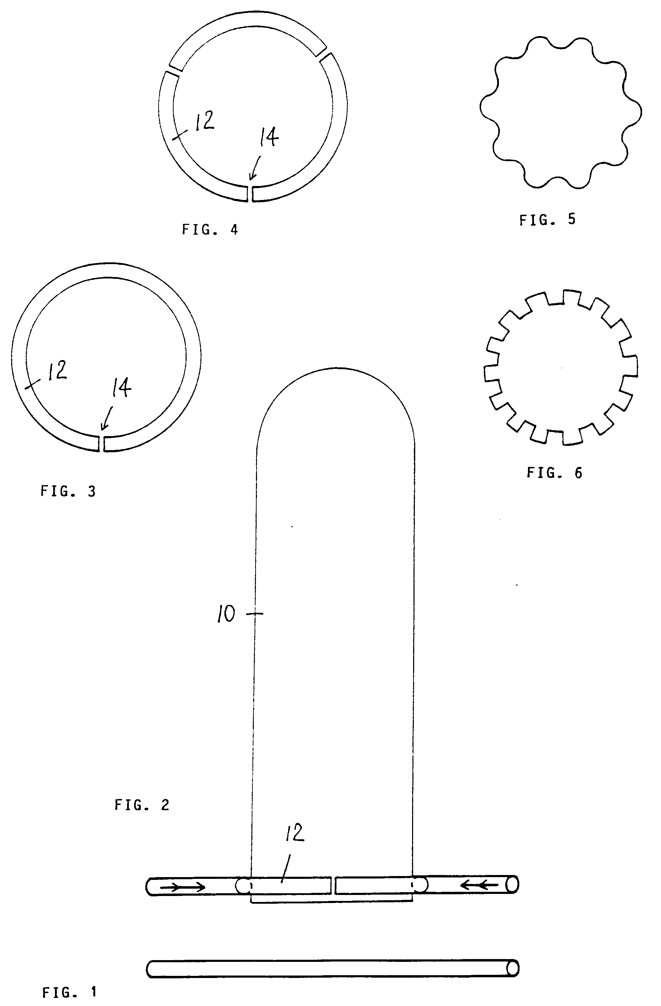

In Fig. 2 ist mit 10 das eigentliche Präservativ bezeichnet, bei dessen Entrollen dank dem mit eingerollten Materialstreifen 12 ein viel dickerer und damit leichter handhabbarer Wulst gebildet worden war als ohne solchen in Ringform gebogenen Materialstreifen. Der Ring 12 ist in Fig. 1 in seiner bei seiner Fertigung entstandenen Form dargestellt, nämlich als langgestrecktes Teil, das zum Beispiel von einem Strang aus Moosgummi abgelängt sein kann. Zum Einrollen des Präservativs werden die Strangenden unter elastischer Deformation aufeinander zu gebogen (Fig. 3), wie durch die Pfeile in Fig. 2 angedeutet. Nach dem Abrollen kehrt das Teil wieder in seine ursprüngliche gestreckte Form zurück und fällt einfach ab. Es versteht sich, daß "langgestreckt" nicht unbedingt "gerade" bedeuten muß; die Streckung muß nur ausreichen, um das Abfallen zu gewährleisten.

Der Ring 12 nach Fig. 4 ist aus drei "gestreckten", in C-Form gebrachten Ringsektoren zusammengefügt, die nach dem Applizieren des Präservativs ebenfalls abfallen. Der Deutlichkeit halber ist in dieser Figur der Spalt 14 zwischen benachbarten Sektoren übertrieben breit dargestellt.

In beiden Ausführungsformen können die Ringelemente aus dünnwandigen, luftgefüllten Schlauchabschnitten bestehen, die besonders gut rollfähig sind.

Die gewellte Ringkontur nach Fig. 4 oder die gekerbte Ringkontur nach Fig. 5 erleichtern bei der Fertigung des erfindungsgemäßen Präservativs dessen Aufrollen in die verkaufsübliche Form.

Es kann bei der Fertigung zweckmäßig sein, den zusammengebogenen Ring gemäß Fig. 2 bzw. die Ringsektoren nach Fig. 3 an das Präservativ mittels eines geeigneten Klebers anzuheften, um das Einrollen zu vereinfachen; ein solcher Kleber müßte aber seine Klebkraft nach kurzer Zeit verlieren, damit der Ring bzw. die Ringteile wunschgemäß abfallen können. Es kann auch zweckmäßig sein, das Einrollen bei der Fertigung dadurch zu erleichtern, daß an die Präservativöffnung eine schmale, nach außen ragende Lippe angeformt wird, die den Ring bzw. die Ringteile untergreift.

Zu allen erwähnten Anwendungsformen ist es zweckmäßig, daß das gemäß der Erfindung zu benutzende Präservativ mindestens in dem vor dem Benutzen freiliegenden Bereich eine Befeuchtung durch ein Gleitmaterial erhält. Dank des stark verdickten Wulstes, welcher vor dem Abrollen verhanden ist, gebildet gemäß der Erfindung, plus dem Gleitmaterial, wird so ein wirklich leichtes Aufsetzen auf die Peniskuppe mit anschließend problemlosem Abrollen des Präservativs garantiert.

## Patentansprüche

1. Präservativ, das auf einen verdickten Ring aus elastischem Material aufgerollt ist, dadurch gekennzeichnet, daß der Ring aufgeschnitten ist und aus einer gestreckten Form in Ringform gebracht ist.

2. Präservativ nach Anspruch 1, dadurch gekennzeichnet, daß der Ring an nur einer Stelle aufgeschnitten ist und aus der gestreckten Form in die Ringform gebogen ist.

3. Präservativ nach Anspruch 1, dadurch gekennzeichnet, daß die erhaltene Ringform aus mehreren Ringsegmenten zusammengesetzt ist aus ebenfalls in gestreckter Form hergestellten Teilstücken, die in C-Form aneinanderliegend einen Ring bilden.

4. Präservativ nach einem der Ansprüche 1 bis 3, dadurch gekennzeichnet, daß das in Ringform gebrachte Material aus einem Elastomermaterial besteht.

5. Präservativ nach einem der vorangehenden Ansprüche, dadurch gekennzeichnet, daß jedes Ringelement ein luftgefüllter Schlauchabschnitt ist.

6. Präservativ nach einem der vorangehenden Ansprüche, dadurch gekennzeichnet, daß das in Ringform gebrachte Material eine gewellte oder mit Einkerbungen versehene Außenkontur aufweist.

7. Präservativ nach einem der Ansprüche 1 bis 6, dadurch gekennzeichnet, daß das Präservativ mindestens in dem vor dem Applizieren freiliegenden Bereich eine Befeuchtung aufweist.
